# EUROPEAN PATENT SPECIFICATION

(11) **EP 0 386 222 B1**
(45) Date of publication and mention of the grant of the patent: **08.02.1995**
(21) Application number: 89910461.6
(22) Date of filing: 06.09.1989
(51) Int. Cl.: C12P 21/00, C12N 15/14

(54) **Process for preparing human serum albumine by fermenting a genetically engineered microorganism in the presence of a polyalkylene compound**
Verfahren zur Herstellung von menschlichem Serumalbumin durch Gärung eines genetisch veränderten Mikroorganismus in Gegenwart eines Polyalkylens
Procédé de préparation de sérum albumine humaine par fermentation d'un microorganisme obtenu par génie génétique en présence d'un polyalkylène

(30) Priority: 07.09.1988 GB 8820951
(43) Date of publication of application: 12.09.1990
(73) Proprietor: Delta Biotechnology Limited, Nottingham NG7 1FD (GB)
(72) Inventor: CLARKE, Peter, Maurice, Radcliffe on Trent Nottinghamshire NG12 (GB); COLLINS, Stephen, Hugh, Radcliff on Trent Nottinghamshire NG12 (GB); MEAD, David, John, Radcliffe on Trent Nottinghamshire NG12 (GB)
(74) Representative: Bassett, Richard
(86) International application number: GB8901046
(87) International publication number: WO9002808

(56) References cited:
- EP-A- 73 646
- EP-A- 262 516
- GENE, vol. 25, 1983; R.J. KLEBE et al., pp. 333-341#
- DIALOG INFORMATION SERVICES, file 5, Biosis, Biosis no. 82023603, KI W.K. et al.; Korean J. appl. Microbiol. bioeng. 14(2), 1986, pp. 125-132

## Description

This invention provides improved fermentation methods and, in particular, a method of increasing the quantity of human serum albumin (hereinafter "albumin") produced by fermentation of a microorganism which is capable of secreting albumin into the medium.

Albumin is a major component of blood plasma and can be used as a plasma or serum substitute to treat burns. haemorrhagic shock and other conditions. Methods have been described whereby albumin can be produced by fermentation of microorganisms into which the gene for albumin has been introduced by recombinant DNA technology (GB-A-2147903).

It is preferable that such organisms are so constructed as to secrete albumin into the surrounding culture fluid because intracellular albumin, in common with many other heterologous proteins, is produced in an inactive, insoluble form from which the native protein can be obtained only with great difficulty (see, for example, M. Latta et al 1987 Bio/Technology 5 1309-1314). In order to maximize the productivity of the manufacturing process it is desirable to grow the microorganisms in a highly agitated aerated fermenter to achieve high concentrations of cells and product. Unfortunately these conditions leave the secreted protein exposed to physical, chemical and enzymatic degradation in the extracellular medium such that the quantity of albumin produced in the fermenter is much less than might have been expected from the performance of the organisms in less intensive conditions.

We have found that the addition of certain chemical reagents is effective in raising the concentration of secreted albumin especially in minimal media (i.e. growth media which do not contain complex organic sources of nitrogen). In particular the addition of certain antifoam agents to levels much greater than those required for normal foam control has this stabilising effect. Effective reagents include polypropylene glycol (average molecular weight 2,000) referred to as PPG 2000 hereafter and "polyricinate" (a condensate of castor oil fatty acids and ethylene oxide produced by Croda Chemicals).

Any suitable polyalkylene polymer or copolymer with another compound may be used, for example polyethylene glycol; polypropylene glycol; polysorbate 80; copolymers of ethylene oxide and propylene oxide; and copolymers of ethylene oxide and/or propylene oxide with other compounds, such as fatty acids, fatty alcohols, sugars and polyols, for example sugar alcohols (e.g. sorbitol), ethylene glycol, pentaerythritol and glycerol.

Polyoxypropylene polymers have the general formula HO-(CH₂-CH(CH₃)-0)ₙ-H. Suitable polyoxyethylene - polyoxypropylene copolymers include those which can be represented as X-(0-(CH₂-CH(CH₃)-0)ₘ-(CH₂-CH₂0)ₙ-H)L where X is a polyol containing l hydroxyl groups, l is 2-6, m is 10-30 and n is 0-10. Another suitable series of compounds uses polyethylene glycol as the polyol, having two available hydroxyl groups, each substitutable by a -0(CH₂-CH(CH₃)-0)ₙ-H group where each n is the same or different.

All of the compounds and classes of compounds referred to above are referred to herein as "polyoxyalkylene compounds". These compounds are usually, but not always, antifoam compounds. If such a compound does not have antifoam properties, then the phrase "in excess of the amount required to suppress undesirable levels of foam" means in an amount greater than zero.

Suitable compounds include those available under the trade designations "Darastil" (for example "Darastil 8231") (Grace Dearborn Ltd, Widnes, Cheshire, U.K.) and Breox FMT30 (Water Management Chemicals Ltd, Kidderminster, Worcs, U.K.)

High cell densities in microbial fermentations are commonly achieved by fed-batch culture. Under these conditions the fermenter is part-full at the start of the process and contains only a small fraction of the carbon substrate. As the fermentation proceeds a concentrated solution of the carbon substrate, and possibly other nutrients, is gradually added. Antifoams are a common process aid in such a process, foam control being achieved by a sensor situated near the top of the fermenter which actuates the addition of antifoam when foam is detected. Since the fermenter is only partially filled at the start, little or no antifoam is added in the early stages of the fermentation and it is only towards the end of the process, as the vessel fills up, that a head of foam is likely to reach and actuate the sensor. Typical usage rates are 0.02-0.2g/l fermentation broth. In contrast, in the process here described, the stabilising agent is added from the start (or at an early stage) of the fermentation. The reagent may be added either in a single dose at or near the start of fermentation or it may be added gradually or intermittently in a series of aliquots through the process. In this way it is possible to maintain approximate proportionality between cell biomass or albumin, on the one hand, and reagent on the other.

One or more suitable stabiliser compounds may be used. Usage rates for this process are such as to yield a concentration of stabiliser (or of total stabilisers, if more than one is used) of between 0.5 and 10g/l (preferably 1-5g/l). Although the use of fed-batch culture is particularly suitable for the production of high cell densities for heterologous protein production, the process is not restricted to this type of fermentation. Use of these reagents at these concentrations is also applicable to the production of albumin in simple batch culture or in continuous culture. In the latter case the reagent can be added continuously or intermittently to maintain a concentration in the desired range of 0.5-10g/l (preferably 1-5g/l).

The use of the stabilisers of the invention has been found to be beneficial with relatively low-yielding strains of yeast. With certain strains of yeast, which give higher yields of albumin, the beneficial effect may be less noticeable if the fermentation protocol is such as to produce high cell densities, but is still detectable if lower cell densities are produced.

The albumin may be any naturally-occurring or modified form of albumin, including fragments thereof, provided that the compound retains at least one functional characteristic of HSA, for example its oncotic or ligand-binding properties or its utility in laboratory media. In particular, the albumin may be the fragment disclosed in EP-A-322 094. Secretion may be mediated by any suitable signal sequence, for example the pre-HSA sequence or the yeast alpha-factor signal sequence.

The microorganism may be any organism (including animal or plant cell cultures) which secretes albumin into the fermentation medium, such as suitable strains of bacteria (for example Bacillus or Strentomyces spp.) or yeasts (such as Saccharomyces cerevisiae or Kluyveromyces lactis). Albumin-secreting yeasts and their fermentation to produce albumin are disclosed in EP-A-322 094 and EP-A-201 239.

### Examples

### Example 1. Stabilisation of albumin in fermentation medium.

A laboratory fermenter was filled to half its nominal working volume with an initial "batch" medium containing 50ml/l of a salts mixture (containing 114g/l KH₂PO₄, 12g/l MgSO₄, 3.0g/l CaCl₂.6H₂O, 2.0g/l Na₂ EDTA; 10ml/l of a trace elements solution containing 3g/l ZnSO₄.7H₂O, 10g/l FeSO₄.7H₂O, 3.2g/l MnSO₄.4H₂O, 79mg/l CuSO₄.5H₂O, 1.5g/l H₃BO₃, 0.2g/l KI, 0.5g/l Na₂MoO₄.2H₂O, 0.56g/l CoCl₂.6H₂O, 75ml/l H₃PO₄; 20g/l sucrose; 50ml/l of a vitamins mixture containing 1.6g/l Ca pantothenate, 1.2g/l nicotinic acid, 12.8g/l m inositol, 0.32g/l thiamine HCl, 0.8g/l pyridoxine HCl and 8mg/l biotin. An equal volume of "feed" medium containing 100ml/l of the salts mixture, 20ml/l of trace elements solution 500g/l sucrose and 100ml/l vitamin solution was held in a separate reservoir connected to the fermenter by a metering pump.

500mg/l albumin was added to the fermenter which was inoculated with Saccharomyces cerevisiae . pH was maintained at 5.7 ± 0.2 by automatic addition of ammonia or sulphuric acid, the temperature was kept at 30°C and the stirrer speed was adjusted to give a dissolved oxygen tension (DOT) of > 20% air saturation at 1 v/v/min air flow rate. When the initial substrate had been consumed, the metering pump was turned on, maintaining a growth rate of approximately 0.15h⁻¹. The pump rate was increased to maintain this growth rate until the stirrer speed reached its maximum value at which point it was not possible to increase the pump rate any further without causing the DOT to fall below 15% air saturation which was the minimum value permitted to occur. PPG 2000 was added in response to a foam sensor. None was added until over 50% of the feed solution had been added. The final level of addition was 0.2g/l. Biomass concentration at the end of the fermentation was 93g/l. About 90% of the albumin initially present was degraded within 24h of the start of the feed.

In a subsequent experiment under otherwise identical conditions PPG was added at the start of fermentation to a level of 1g/l. The progress of the fermentation was very similar, reaching a final dry weight of 94g/l, but in this case albumin degradation was subsequently reduced so that at the end of the fermentation about 60% of the albumin originally present was undegraded.

### Example 2. Increasing Concentration of Secreted Albumin

A series of experiments were set up using the fed batch protocol described above with the modification that in this case the pump rate was increased automatically using a computer control system which also monitored respiratory quotient (RQ) so that the feed rate was reduced if RQ exceeded 1.2. This procedure avoids possible yield loss by the "Crabtree" affect. In all cases fermenters were inoculated with Saccharomyces cerevisae containing a plasmid which codes for secreted albumin production. The following protocols were used for antifoam addition with the following results.

### Example 2A (comparative example)

PPG 2000 added solely in response to foam sensor. Nil at fermentation start, increasing to 0.1g/l after 10% of the feed was added, then rising to a final level of 0.2g/l during the last 50% of the feed. The final biomass was 84g/l, and the albumin level was noted on an arbitrary scale to be 1.0 unit.

### Example 2B

Polyricinate added to 1.5g/l at start of fermentation, increasing further to 2.2g/l after 35% of feed added with further increases to maintain a level of 2.5g/l when feed was nearly complete. A further addition after the feed was over raised the final level to 3.5g/l. Final biomass 83g/l, albumin level 3.81 units.

### Example 2C

PPG 2000 added to an initial value of 1g/l with further additions after 50% and 80% of the feed had been added to maintain this concentration. Final biomass 86g/l, albumin concentration 4.2 units.

### Example 3. Increasing Concentration of Secreted Albumin (second case)

A series of the fed-batch fermentations were set up according to the procedure of Example 2 except that a different strain of S. cerevisiae (but still containing the plasmid for secreted albumin) was used. Antifoams were added according to the protocol of Example 2C with the following results.

| Antifoam | Biomass (g/l) | Albumin Concentration (arbitrary units) |
|---|---|---|
| Arachis oil | 95.4 | 1.0 |
| Crill 1 | 86.8 | 0.8 |
| Breox FMT30 | 81.8 | 3.2 |
| Darastil 8231 | 81.6 | 2.5 |

It is apparent that the polyalkene oxide derived antifoams (Darastil 8231 and Breox FMT30) give higher albumin yields than the others.

## Claims

1. A process for preparing human serum albumin by fermenting an albumin-secreting microorganism in a suitable medium such that albumin is secreted into the medium, characterised in that a polyoxyalkylene compound is added to the medium, wherein, if the polyoxyalkylene compound is an antifoam compound, the polyoxyalkylene compound is so added as to give an average level of more than 0.2g/l.

2. A process according to Claim 1 wherein the polyoxyalkylene compound is so added as to give an average level of 0.5 to 10g/l.

3. A process according to Claim 2 wherein the polyoxyalkylene compound is so added as to give an average level of 1.0 to 5.0g/l.

4. A process according to any of the previous claims wherein the polyoxyalkylene compound is an antifoam compound.

5. A process according to any one of the preceding claims wherein the polyoxyalkylene compound is a polypropylene glycol; a condensate of castor oil fatty acids and ethylene oxide; a copolymer of propylene oxide and ethylene oxide with a polyol; or a copolymer of propylene oxide and polythylene glycol.

6. A process according to any one of the preceding claims wherein the microorganism is a yeast.

7. A process according to Claim 6 wherein the yeast is Saccharomyces cerevisiae.

8. A process according to any one of the preceding claims wherein the medium is a minimal medium.

## Patentansprüche

1. Verfahren zur Herstellung von Humanserumalbumin durch Fermentation eines albuminabscheidenden Mikroorganismus in einem geeigneten Medium dergestalt, daß Albumin in das Medium abgeschieden wird, dadurch gekennzeichnet, daß dem Medium eine Polyoxyalkylenverbindung zugesetzt wird, wobei die Polyoxyalkylenverbindung im Falle, daß sie ein Antischaummittel ist, in einer solchen Menge zugesetzt wird, daß ein durchschnittlicher Gehalt von mehr als 0,2 g/l errreicht wird.

2. Verfahren nach Anspruch 1, wobei die Polyoxyalkylenverbindung in einer solchen Menge zugesetzt wird, daß ein durchschnittlicher Gehalt von 0,5 - 10 g/l erreicht wird.

3. Verfahren nach Anspruch 2, wobei die Polyoxyalkylenverbindung in einer solchen Menge zugesetzt wird, daß ein durchschnittlicher Gehalt von 1,0 - 5,0 g/l erreicht wird.

4. Verfahren nach einem der vorhergehenden Ansprüche, wobei die Polyoxyalkylenverbindung aus einem Antischaummittel besteht.

5. Verfahren nach einem der vorhergehenden Ansprüche, wobei die Polyoxyalkylenverbindung aus einem Polypropylenglykol, einem Kondensat von Rizinusölfettsäuren und Ethylenoxid, einem Copolymer von Propylenoxid und Ethylenoxid mit einem Polyol oder einem Copolymer von Propylenoxid und Polyethylenglykol besteht.

6. Verfahren nach einem der vorhergehenden Ansprüche, wobei der Mikroorganismus aus einer Hefe besteht.

7. Verfahren nach Anspruch 6, wobei die Hefe aus Saccharomyces cerevisiae besteht.

8. Verfahren nach einem der vorhergehenden Ansprüche, wobei das Medium aus einem Minimalmedium besteht.

## Revendications

1. Procédé pour la préparation de sérum albumine humaine par fermentation d'un microorganisme sécrétant de l'albumine dans un milieu convenable tel que l'albumine est sécrétée dans le milieu, caractérisé en ce qu'un polyoxyalkylène est ajouté au milieu, dans lequel, si le polyoxyalkylène est un composé antimousse, le polyoxyalkylène est incorporé de façon à donner une concentration moyenne supérieure à 0,2 g/litre.

2. Procédé suivant la revendication 1, dans lequel le polyoxyalkylène est ajouté de façon à donner une concentration moyenne de 0,5 à 10 g/litre.

3. Procédé suivant la revendication 2, dans lequel le polyoxyalkylène est ajouté de façon à donner une concentration moyenne de 1,0 à 5,0 g/litre.

4. Procédé suivant l'une quelconque des revendications précédentes, dans lequel le polyoxyalkylène est un composé antimousse.

5. Procédé suivant l'une quelconque des revendications précédentes, dans lequel le polyoxyalkylène est un polypropylène glycol, un condensat d'acides gras d'huile de ricin et d'éthylène, un copolymère d'oxyde de propylène et d'oxyde d'éthylène avec un polyol, ou un copolymère d'oxyde de propylène et de polyéthylène glycol.

6. Procédé suivant l'une quelconque des revendications précédentes, dans lequel le microorganisme est une levure.

7. Procédé suivant la revendication 6, dans lequel la levure est Saccharomyces cerevisiae.

8. Procédé suivant l'une quelconque des revendications précédentes, dans lequel le milieu est un milieu minimum.
